# EUROPEAN PATENT APPLICATION

(11) **EP 4 779 067 A1**
(43) Date of publication of application: **22.07.2026**
(21) Application number: 24865513.6
(22) Date of filing: 12.09.2024
(51) Int. Cl.: D01F 4/00, A61L 27/24, D01D 5/06

(54) **COLLAGEN YARN, METHOD FOR PRODUCING SAME, AND BIOMATERIAL**

(30) Priority: 15.09.2023 JP 2023150114
(71) Applicant: Nitta Gelatin Inc., Osaka 556-0022 (JP); National University Corporation Hokkaido University, Hokkaido 060-0808 (JP)
(72) Inventor: YUNOKI, Shunji, Sapporo-shi, Hokkaido 060-0808 (JP); KONDO, Eiji, Sapporo-shi, Hokkaido 060-0808 (JP); HIRAOKA, Yosuke, Yao-shi, Osaka 581-0024 (JP); MANDAI, Yoshinobu, Yao-shi, Osaka 581-0024 (JP); KISHIMOTO, Masanori, Yao-shi, Osaka 581-0024 (JP)
(74) Representative: Vossius & Partner Patentanwälte Rechtsanwälte mbB
(86) International application number: PCT/JP2024/032689
(87) International publication number: WO 2025/058013

(57) **Abstract**

A collagen yarn comprises collagen nanofibrils, wherein the collagen yarn has a diameter from 10 µm to 50 µm, the collagen yarn has a total length of 2×10⁵ times or more the diameter, the collagen nanofibrils are arranged in a highly-aligned state along a longitudinal direction of the collagen yarn, the highly-aligned state is a state where birefringence measurement of a hydrated collagen yarn yields a refractive index difference of 4.3 × 10⁻⁴ or more, and the hydrated collagen yarn is obtained by immersing the collagen yarn in a neutral phosphate buffer.

## Description

### TECHNICAL FIELD

The present invention relates to a collagen yarn, a method of producing the same, and a biological material.

### BACKGROUND ART

As disclosed by Japanese Patent Laying-Open No. 2019-085373 (PTL 1) and the like, development of artificial collagen yarns mimicking biological collagen fibers has been actively pursued. Artificial tendon made of a bundle of artificial collagen yarns and membrane material made by weaving artificial collagen yarns, among others, can be useful as cell scaffold material, and expectations are growing on the use of the artificial tendon and the membrane material as medical material.

### CITATION LIST

### PATENT LITERATURE

PTL 1: Japanese Patent Laying-Open No. 2019-085373

### NON PATENT LITERATURE

NPL 1: Haynl C et al. NANO LETTERS 2016:16:5917-5922
NPL 2: Paten J A et al. ACS Nano 2016:10:5027-5040

### SUMMARY OF INVENTION

### TECHNICAL PROBLEM

By using artificial collagen yarns that have at least four characteristics listed below, it can be possible to artificially mimic biological collagen fibers.
1) Safe for living body
2) Nearly as thin as biological collagen fibers (with a diameter of about 20 µm)
3) Collagen nanofibrils constituting the artificial collagen yarns are aligned all the way into the interior of each artificial collagen yarn, preferably into the core portion
4) Long enough for preparation of artificial tendon and membrane material

In the highly-oriented collagen fibrils bundle disclosed by PTL 1, the fibrils are aligned all the way into the interior of each artificial collagen yarn but the diameter is about 100 µm at the thinnest portion, and for this and other reasons, further improvement is required for mimicking biological collagen fibers. The method disclosed by Haynl C et al. NANO LETTERS 2016:16:5917-5922 (NPL 1) uses a combination of a traditional wet spinning technique and a microchannel technique and is capable of continuously producing artificial collagen yarns having a diameter of about 4 µm. However, it is known that wet spinning can make collagen fibrils aligned at the surface of the yarn but cannot make collagen fibrils aligned inside the yarn. As for the flow-induced crystallization of collagen disclosed by Paten J A et al. ACS Nano 2016:10:5027-5040 (NPL 2), due to the nature of the production process, the collagen nanofibrils can be aligned all the way into the interior and the core portion of artificial collagen yarns, but it is difficult to ensure the sufficient length for preparing an artificial tendon and a membrane material. Hence, thus far, there have been no artificial collagen yarns capable of artificially mimicking biological collagen fibers, and development thereof has been desired.

In light of the above-described circumstances, the present invention aims at providing collagen yarns capable of artificially mimicking biological collagen fibers, a method of producing the same, and a biological material.

### SOLUTION TO PROBLEM

The inventors of the present invention have conducted intensive research to solve the above-mentioned problems and, as a result, the present invention has now been completed. The inventors of the present invention conceived discharging a raw material that is made of a high-concentration (3.0 mass% to 5.0 mass%, for example) neutral collagen solution, through a thin channel (with an inner diameter from 100 µm to 300 µm, for example), at a high shear rate (100 s⁻¹ or more, for example), toward a coagulation bath filled with a high-concentration (70 volume% to 95 volume%) ethanol solution, to prepare collagen yarns. According to the findings of the inventors, each collagen yarn obtained by this preparation method has a diameter of 50 µm or less, the constituent collagen nanofibrils are aligned all the way into the interior of the collagen yarn, preferably into the core portion, and the length is 2×10⁵ times or more the diameter; and thus, the present invention has been devised.

The present invention has the following characteristics.
[1] A collagen yarn according to the present invention is
   a collagen yarn comprising collagen nanofibrils, wherein
   the collagen yarn has a diameter from 10 µm to 50 µm,
   the collagen yarn has a total length of 2×10⁵ times or more the diameter,
   the collagen nanofibrils are arranged in a highly-aligned state along a longitudinal direction of the collagen yarn,
   the highly-aligned state is a state where birefringence measurement of a hydrated collagen yarn yields a refractive index difference of 4.3 ×10⁻⁴ or more, and
   the hydrated collagen yarn is obtained by immersing the collagen yarn in a neutral phosphate buffer.
[2] Preferably, the collagen nanofibrils are made of atelocollagen.
[3] Preferably, the total length is 1×10⁶ times or more the diameter.
[4] A method of producing a collagen yarn according to the present invention comprises:
   preparing a neutral collagen solution containing collagen in a concentration from 3.0 mass% to 5.0 mass%; and
   obtaining a collagen yarn by discharging the neutral collagen solution into an ethanol solution having a concentration from 70 volume% to 95 volume%, wherein
   the collagen is atelocollagen, and
   in the obtaining a collagen yarn, the neutral collagen solution is discharged through a channel having an inner diameter from 100 µm to 300 µm into the ethanol solution at a shear rate from 100 s⁻¹ to 1500 s⁻¹.
[5] Preferably, the method of producing a collagen yarn further comprises drawing the collagen yarn by taking up the collagen yarn at a take-up speed of 1.5 times to 4.0 times a discharging linear speed.
[6] Preferably, the neutral collagen solution contains the collagen in a concentration from 4.5 mass% to 5.0 mass%.
[7] Preferably, the shear rate is from 300 s⁻¹ to 1000 s⁻¹.
[8] A biological material according to the present invention contains the collagen yarn.
[9] Preferably, the biological material is an artificial tendon or a membrane material.

### ADVANTAGEOUS EFFECTS OF INVENTION

The present invention has made it possible to provide collagen yarns capable of artificially mimicking biological collagen fibers, a method of producing the same, and a biological material.

### BRIEF DESCRIPTION OF DRAWINGS

Fig. 1 is a scanning electron microscope image of a cross section of a collagen yarn according to the present embodiment, cut along the axial direction.
Fig. 2 is a descriptive view for illustrating birefringence measurement of a hydrated collagen yarn performed by line analysis for the purpose of evaluating the highly-aligned state of collagen nanofibrils constituting a collagen yarn according to the present embodiment.
Fig. 3 is an image of the appearance of a biological material (an artificial tendon) made of collagen yarns according to the present embodiment, captured with a digital camera.
Fig. 4 is a flowchart illustrating an example of a method of producing a collagen yarn according to the present embodiment.
Fig. 5 is a schematic view of a production apparatus used for preparing a collagen yarn according to the present embodiment.
Fig. 6 is another schematic view of a production apparatus used for preparing a collagen yarn according to the present embodiment.

### DESCRIPTION OF EMBODIMENTS

In the following, an embodiment of the present invention (hereinafter also referred to as "the present embodiment") will be described in further detail. In the present specification, the expression "(from) A to B" means the upper limit and the lower limit of a range (that is, it means not less than A and not more than B), and when A is not accompanied by unit expression and B is accompanied by unit expression, the unit of A is the same as the unit of B. The term "alignment (or aligned)" means a state where the axes of collagen nanofibrils formed of an orderly array of collagen molecules are arranged in order. In some prior art literatures, the term "orientation", which means that not only the axes of the collagen nanofibrils are arranged in order but also the collagen nanofibrils are arranged in the same directions, is used as a synonym of "alignment (or aligned)"; however, in the present specification, these two terms are distinguished from each other.

In the present specification, the "interior" of a collagen yarn means the entire body of the collagen yarn except the surface. The "core portion" of a collagen yarn refers to the central portion of the collagen yarn that appears in a cut surface of the collagen yarn cut perpendicularly to the axial direction, and specifically, it means the central portion extending along the axial direction.

### [Collagen Yarn]

A collagen yarn according to the present embodiment is a collagen yarn containing collagen nanofibrils. The collagen yarn has a diameter from 10 µm to 50 µm. The collagen yarn has a total length of 2×10⁵ times or more the diameter. The collagen nanofibrils are arranged in a highly-aligned state along a longitudinal direction of the collagen yarn. The highly-aligned state is a state where birefringence measurement of a hydrated collagen yarn yields a refractive index difference of 4.3 × 10⁻⁴ or more. The hydrated collagen yarn is obtained by immersing the collagen yarn in a neutral phosphate buffer. A collagen yarn having these characteristics is capable of artificially mimicking a biological collagen fiber. Hence, there is a possibility that the above-mentioned collagen yarn can be used as a biological material such as an artificial tendon or a membrane material.

### <Collagen Nanofibrils>

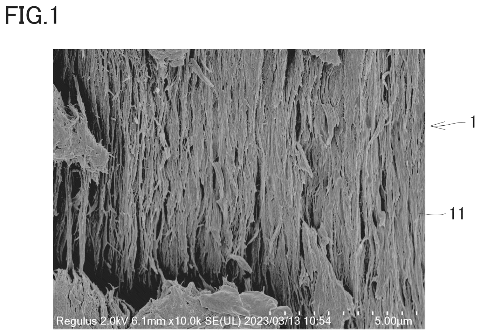

Fig. 1 is a scanning electron microscope image of a cross section of a collagen yarn according to the present embodiment, cut along the axial direction. Referring to Fig. 1, a collagen yarn 1 contains collagen nanofibrils 11. Collagen nanofibril 11 refers to a self-assembled body of numerous collagen molecules assembled in an array in a moistened neutral environment, having a yarn-like appearance with a total thickness of about 100 nm. Collagen nanofibrils 11 can be obtained from collagen that have fibril-forming capability (fibril-forming collagen). As the fibril-forming collagen, type I collagen (a collagen that forms bones, skin, tendons, and ligaments), type II collagen (a collagen that forms cartilage), type III collagen (contained in living tissue made of type I collagen), and the like are preferably used. The fibril-forming collagen can be obtained by extraction and purification from living tissue by conventional procedures, or it can be a commercially available product (under the trade name "Collagen BM" manufactured by Nitta Gelatin Inc., for example). The fibril-forming collagen can be solely composed of one type of collagen, or can be composed of a mixture of a plurality of types of collagens. The animal species from which the fibril-forming collagen is obtained is not particularly limited. However, from the viewpoint of use as a biological material, the collagen is preferably derived from mammals such as pigs and cows.

Preferably, collagen nanofibrils 11 are made of atelocollagen. In this case, it is possible to provide a collagen yarn that has low antigenicity and high biocompatibility. "Atelocollagen" refers to telopeptide-free collagens prepared by enzymatically degrading and removing telopeptides at the N terminus and the C terminus of each collagen molecule, with a protease such as pepsin, chymosin, cathepsin D, and/or renin. As the atelocollagen constituting the collagen nanofibrils, in particular, a telopeptide-free collagen derived from a mammal and approved for use as a raw material of a medical instrument is preferable, among the above-mentioned telopeptide-free collagens, and a telopeptide-free collagen derived from pig skin is more preferable.

### <Diameter>

The diameter of collagen yarn 1 is from 10 µm to 50 µm. From the viewpoint of use as a biological material such as an artificial tendon or a membrane material, the diameter of collagen yarn 1 is preferably from 10 µm to 40 µm, more preferably from 20 µm to 30 µm. When collagen yarn 1 has a diameter of less than 10 µm, there is a possibility that it can be too thin, not strong enough, and poor in take-up properties to, for instance, break during the taking-up process. When collagen yarn 1 has a diameter of more than 50 µm, there is a possibility that it can be too thick and less capable of mimicking the function (for example, cell scaffold function) of a biological collagen fiber. The diameter of collagen yarn 1 can be determined by performing image analysis of an image obtained by scanning electron microscopy of a cross section of collagen yarn 1 to determine a cross-sectional area, and regarding the diameter of a circle whose area is the same as the cross-sectional area (an equivalent circular diameter) as the diameter of collagen yarn 1.

### <Total Length>

The total length of collagen yarn 1 is 2×10⁵ times or more the diameter. In this case, one collagen yarn 1 can be long enough for preparing a biological material, namely, an artificial tendon and a membrane material. From the viewpoint of preparing an artificial tendon and a membrane material with one collagen yarn 1, the total length is preferably 1×10⁶ times or more the diameter. Specifically, the total length of collagen yarn 1 is preferably 10 m or more, more preferably 100 m or more, most preferably 200 m or more. The upper limit to the total length of collagen yarn 1 is not particularly limited. From the viewpoint of preparing an artificial tendon and a membrane material, for instance, the total length of collagen yarn 1 is ideally 1000 m. The total length of collagen yarn 1 can be determined by measuring the length of the collagen yarn obtained by a below-described method of producing a collagen yarn, that is taken up without breaking during the taking-up process.

### <Highly-Aligned State>

Collagen nanofibrils 11 are arranged in a highly-aligned state along the longitudinal direction of collagen yarn 1. The highly-aligned state is a state where birefringence measurement of a hydrated collagen yarn yields a refractive index difference of 4.3×10⁻⁴ or more. The hydrated collagen yarn is obtained by immersing collagen yarn 1 in a neutral phosphate buffer. In the present specification, the "highly-aligned state" means that collagen nanofibrils 11 constituting collagen yarn 1 are aligned all the way into at least the interior of collagen yarn 1, preferably into the core portion. As long as collagen fibrils can be stably present in the neutral phosphate buffer, there is no particular limitation on the pH, the ion concentration, the amount of sodium chloride to be added, and the like. However, from the viewpoint of reducing salt deposition and forming the hydrated collagen yarns hard enough for easy handling, the neutral phosphate buffer is preferably a low-concentration (for example, 10 mM) sodium hydrogen phosphate solution not containing sodium chloride and adjusted to pH7.

One of the reasons why the highly-aligned state exhibits a refractive index difference of 4.3×10⁻⁴ or more is as follows. The refractive index difference is, as described below, a value obtained by dividing the optical path difference that occurs at the time when an inherently-polarized light of a certain type passes through the interior of the hydrated collagen yarn, by the thickness of the hydrated collagen yarn. The greater the value of the refractive index difference is, the more collagen nanofibrils 11 in the hydrated collagen yarn have their axes arranged in order. Moreover, the above-mentioned numerical value of the refractive index difference, 4.3×10⁻⁴ or more, is obtained only when collagen nanofibrils 11 are aligned all the way into at least the interior of collagen yarn 1, preferably into the core portion, as checked in a cross-sectional image of the hydrated collagen yarn.

In the above-mentioned case, even when the surface of collagen yarn 1 is digested or degraded with an enzyme and the like present in the living body and thereby the interior is exposed, collagen yarn 1 is still recognizable by cells due to the above-mentioned highly-aligned state and is still capable of functioning as a cell scaffold material. In the following, a description will be given of the birefringence measurement of a hydrated collagen yarn performed for the purpose of evaluating the highly-aligned state of collagen nanofibrils 11.

### (Birefringence Measurement)

In the present embodiment, the highly-aligned state of collagen nanofibrils 11 is a state where, as described above, birefringence measurement of a hydrated collagen yarn yields a refractive index difference of 4.3 × 10⁻⁴ or more. The birefringence measurement refers to determining a phase difference of light (retardation; the unit is nm) that occurs when circularly polarized light enters into the hydrated collagen yarn, with the use of a known two-dimensional birefringence measurement apparatus (under the trade name "WPA-200" manufactured by Photonic Lattice, for example). The refractive index difference can be determined by dividing the phase difference by the thickness of the hydrated collagen yarn (namely, the distance the light traveled).

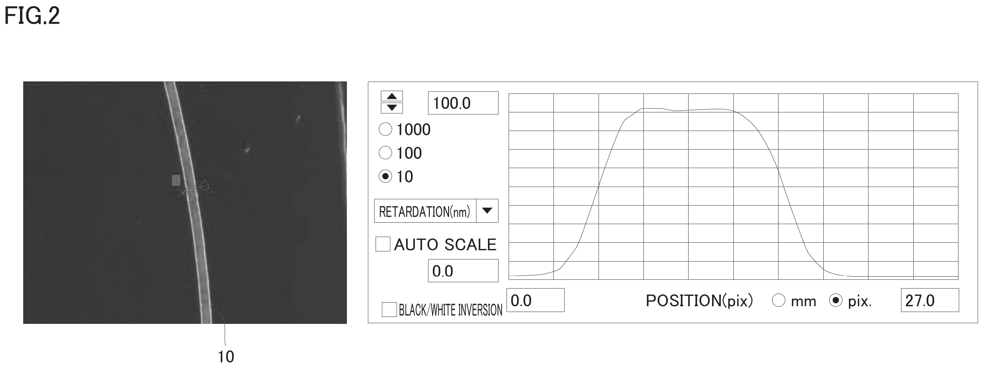

Due to the fact that a cross section of the hydrated collagen yarn is almost a perfect circle, the refractive index difference can be calculated by line analysis of a two-dimensional phase difference image. In a graph where the horizontal axis represents the position in a direction perpendicular to the axial direction of the hydrated collagen yarn (hereinafter also referred to as "the width direction") and the vertical axis represents the phase difference, the phase difference obtained by line analysis looks like an upward-convex arc. In this case, the phase difference data for use in calculation of the refractive index difference is obtained from the central 50% of the upward-convex arc, and for the sake of convenience, the width of the hydrated collagen yarn separately measured with a known digital microscope and multiplied by 0.95 can be used as the thickness for use in the calculation. This is because the thickness of the hydrated collagen yarn decreases sharply at the edge of the upward-convex arc but the thickness at the central 50% is almost the same as the diameter multiplied by 0.95. Furthermore, the refractive index difference can be determined by averaging five numerical values obtained by the above-mentioned line analysis performed at freely-selected five positions of the hydrated collagen yarn. For instance, when the upward-convex arc is formed over 0 to 8 pixels on the horizontal axis, the "central 50% of the upward-convex arc" means the portion (width) over the central 4±2 pixels (see Fig. 2).

Fig. 2 is a descriptive view for illustrating birefringence measurement of a hydrated collagen yarn performed by line analysis for the purpose of evaluating the highly-aligned state of collagen nanofibrils constituting a collagen yarn according to the present embodiment. Fig. 2 shows an image (left) of birefringence measurement of a hydrated collagen yarn 10 performed by line analysis, as well as a graph (right) where the horizontal axis represents the position in the width direction of hydrated collagen yarn 10 determined by the birefringence measurement and the vertical axis represents the phase difference at each position. The refractive index difference of hydrated collagen yarn 10 shown in Fig. 2 can be calculated from the graph as well as the thickness of hydrated collagen yarn 10, and it is 5.88×10⁻⁴.

Herein, hydrated collagen yarn 10 is obtained by hydrating collagen yarn 1 in a neutral phosphate buffer and then introducing a chemical crosslink thereinto. Specifically, hydrated collagen yarn 10 is prepared as follows: until the swelling of collagen yarn 1, or the swelling of the collagen yarn in ethanol solution before dried, reaches saturation, the collagen yarn is immersed in an excess amount of neutral (pH6.5 to pH7.5) phosphate buffer; and thereto, 1-ethyl-3-[3-(dimethylamino)propyl]carbodiimide (EDC) is added to introduce a chemical crosslink into the collagen. Although the EDC concentration of the phosphate buffer does not affect the birefringence measurement, from the viewpoint of stabilizing the collagen yarn and ensuring sufficient flexibility not to become brittle at the time of handling, the EDC concentration is preferably within the range of 10 mM to 200 mM. Prior to the birefringence measurement, it is preferable to transfer the hydrated collagen yarn 10 again into an excess amount of phosphate buffer to remove a crosslinking agent and a reaction by-product remaining in the interior of the yarn. EDC is suitable as a collagen crosslinking agent for optical analysis because it does not stain collagen or enter into the gaps between collagen molecules.

The refractive index difference is preferably 5.0×10⁻⁴ or more, more preferably 7.0×10⁻⁴ or more. In this case, collagen nanofibrils 11 constituting collagen yarn 1 are arranged in a further excellent highly-aligned state in which they are aligned all the way into the interior of collagen yarn 1, especially into the core portion, and therefore collagen nanofibrils 11 can be effective as a cell scaffold material. When the refractive index difference is less than 4.3×10⁻⁴, collagen nanofibrils 11 may not be aligned all the way into the interior of collagen yarn 1. The upper limit to the refractive index difference is not particularly limited, and, for example, it may be 12×10⁻⁴. Realistically, the refractive index difference of a collagen yarn obtained by a below-described method of producing a collagen yarn is 12×10⁻⁴ or less.

### <Functions and effects>

As described above, the collagen yarn according to the present embodiment can have the above-mentioned characteristics 1) to 4), and with them, there is a possibility that it can artificially mimic a biological collagen fiber. More specifically, the collagen yarn can be safe for living body because it is made of atelocollagen. The collagen yarn can have a diameter close to the diameter of biological collagen fibers, which is 20 µm. The collagen yarn can have the above-mentioned highly-aligned state where the collagen nanofibrils are aligned all the way into the interior of the collagen yarn, preferably into the core portion. Furthermore, the total length of the collagen yarn can be long enough for preparing an artificial tendon and a membrane material.

### [Biological Material]

A biological material according to the present embodiment contains the above-mentioned collagen yarns. The biological material having this characteristic, when the collagen yarns artificially mimic biological collagen fibers, can function as a cell scaffold material in the living body. Especially because it can thus function as a cell scaffold material, the biological material is preferably an artificial tendon or a membrane material. Hence, the biological material is expected to be useful in the field of medical care and the like, as an artificial tendon or a membrane material.

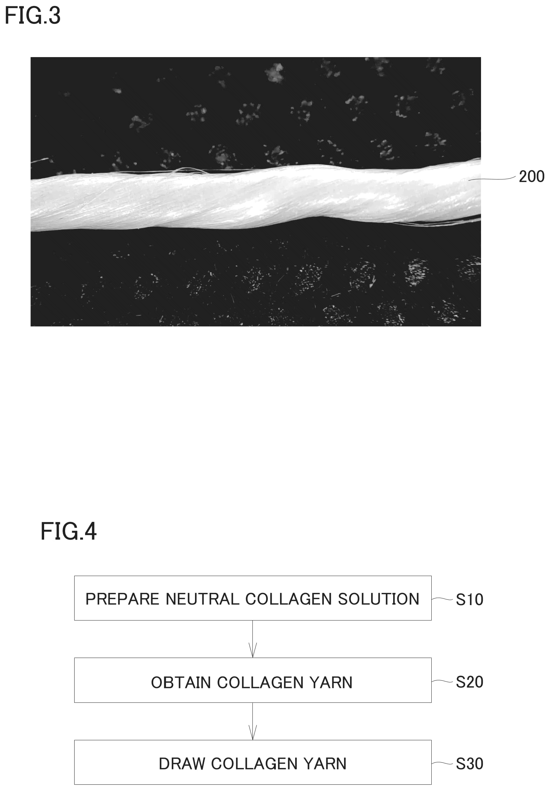

Fig. 3 is an image of the appearance of a biological material (an artificial tendon) made of the collagen yarns according to the present embodiment, captured with a digital camera. Referring to Fig. 3, a biological material made of the collagen yarns can form an artificial tendon 200, based on the above-mentioned characteristics 1) to 4). Artificial tendon 200 illustrated in Fig. 3 is an example of a bundle (with a length of 55 mm and a cross sectional diameter of 1.7 mm) formed of 2000 collagen yarns each having a diameter of 26.8±1.5 µm and a total length of 110 m (more specifically, collagen yarns of Sample 6 in Examples described below). The length and the cross sectional diameter of the artificial tendon can be adjusted to certain values by changing the diameter and the number of the collagen yarns in the bundle. Furthermore, when the biological material made of the collagen yarns is a membrane material (not illustrated in the drawings), the membrane material can be formed by weaving the collagen yarns, for example.

### [Method of Producing Collagen Yarn]

The method of producing the collagen yarn is not particularly limited as long as it can produce a yarn having the above-mentioned characteristics. It should be noted that by using the production method described below, it is possible to produce the above-mentioned collagen yarn with a good yield. More specifically, the method of producing a collagen yarn according to the present embodiment includes preparing a neutral collagen solution containing collagen in a concentration from 3.0 mass% to 5.0 mass%, and obtaining a collagen yarn by discharging the neutral collagen solution into an ethanol solution having a concentration from 70 volume% to 95 volume%. The collagen is atelocollagen. In the obtaining a collagen yarn, the neutral collagen solution is discharged through a channel having an inner diameter from 100 µm to 300 µm into the ethanol solution at a shear rate from 100 s⁻¹ to 1500 s⁻¹. In addition, preferably, the method of producing a collagen yarn further includes drawing the collagen yarn by taking up the collagen yarn at a take-up speed of 1.5 times to 4.0 times the discharging linear speed. By taking up the collagen yarn at a speed faster than the discharging linear speed, the collagen solution is drawn and formed into fibrils (becomes coagulated) immediately after discharged into the ethanol solution, and thin yarns are produced with even higher alignment of fibrils. By the method of producing a collagen yarn having such characteristics, it is possible to obtain collagen yarns that are capable of artificially mimicking biological collagen fibers.

More specifically, the above-mentioned production method preferably has steps illustrated in the flowchart given in Fig. 4, for example. Fig. 4 is a flowchart illustrating an example of a method of producing a collagen yarn according to the present embodiment. Referring to Fig. 4, the method of producing a collagen yarn includes Step S10 (a first step, a preparation step) to prepare a neutral collagen solution containing collagen in a concentration from 3.0 mass% to 5.0 mass%, and Step S20 (a second step, a collagen yarn obtaining step) to obtain a collagen yarn by discharging the neutral collagen solution into an ethanol solution having a concentration from 70 volume% to 95 volume%. Referring to Fig. 4, preferably, the above-mentioned method of producing a collagen yarn further comprises Step S30 (a third step, a collagen yarn drawing step) to draw the collagen yarn by taking up the collagen yarn at a take-up speed of 1.5 times to 4.0 times the discharging linear speed.

In the present specification, the "neutral collagen solution" refers to a neutral solvent (for example, a salt-containing solvent such as phosphate-buffered physiological saline) in which collagen molecules are dissolved without forming fibrils. Therefore, the neutral collagen solution is differentiated from a dispersion that is also based on a neutral solvent but cloudy and whitish with the collagen molecules present in the form of fibrils and dispersed in the solvent. The neutral collagen solution can be slightly turbid due to irreversible fibril formation of some of the collagen that can occur during preparation of the solution, but it does not affect the present invention as long as the optical density at a wavelength of 600 nm and an optical path length of 10 mm does not exceed 0.5. Furthermore, the neutral collagen solution is also differentiated from an acidic solvent in which collagen molecules are dissolved. For instance, the pH of the neutral collagen solution is preferably from 6.0 to 9.0.

As believed by the inventors of the present invention, the reason why the above-mentioned production method can produce collagen yarns capable of artificially mimicking biological collagen fibers is as follows. Firstly, the neutral collagen solution contains collagen in a high concentration from 3.0 mass% to 5.0 mass% (preferably from 4.5 mass% to 5.0 mass%). The "high concentration" means the highest concentration, or the vicinity thereof, in which collagen in the neutral solvent (even though contained in a large amount) does not form fibrils to become deposited at a liquid temperature of 10°C or less. It should be noted that the inventors of the present invention found that the concentration in which collagen in the neutral solvent maintains its molecule-dispersed state is about 5.0 mass%. In other words, the above-mentioned neutral collagen solution is an almost saturated solution for collagen molecules. Therefore, when the collagen molecules in the neutral collagen solution are discharged into a coagulation bath filled with ethanol solution in the collagen yarn obtaining step S20, dehydration (removal of the neutral solvent, particularly water) is caused by ethanol and thereby fibrils are formed in an instant. As a result, the collagen molecules in the neutral collagen solution can become deposited in the form of collagen yarns made of collagen nanofibrils each of which is formed of a bundle of fibrillar collagen molecules. In addition, the neutral collagen solution is discharged through a narrow channel having an inner diameter from 100 µm to 300 µm at a high shear rate. At the time of being discharged from the channel, the collagen molecules in the neutral collagen solution are aligned in the flowing direction.

That is, according to the above-mentioned production method, at the time when they are firstly discharged through the thin channel into the coagulation bath, the collagen molecules in the neutral collagen solution are aligned in one direction. Furthermore, before the alignment in the flowing direction loses its shape, the collagen molecules in the coagulation bath form fibrils, namely, the collagen molecules form bundles (collagen nanofibrils), and then the collagen nanofibrils are spun into collagen yarns. In this case, the collagen nanofibrils can be arranged in a highly-aligned state all the way into the interior of the collagen yarn, preferably into the core portion. With the inner diameter of the channel and the shear rate properly adjusted, the collagen yarn can have a desired diameter. According to the above-mentioned production method, in principle, the length of the collagen yarn to be obtained is not limited. In addition, by using atelocollagen as the collagen molecules, for example, it is possible to obtain collagen yarns that are safe for living body. As described so far, it is conceivable that the above-mentioned production method can produce collagen yarns capable of artificially mimicking biological collagen fibers.

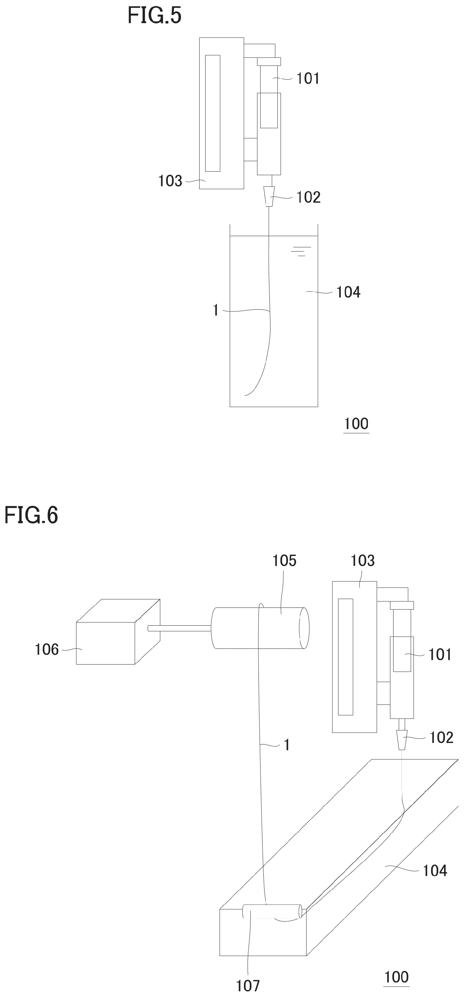

In the following, referring to Fig. 4, Fig. 5, and Fig. 6, a description will be given of the steps of the above-mentioned method of producing a collagen yarn as well as the general outline of a production apparatus used for preparing a collagen yarn in the above-mentioned production method. Fig. 5 is a schematic view of a production apparatus used for preparing a collagen yarn according to the present embodiment. Fig. 6 is another schematic view of a production apparatus used for preparing a collagen yarn according to the present embodiment. According to the above-mentioned method of producing a collagen yarn, the collagen yarn obtaining step S20 is implemented with the use of, for example, the production apparatus illustrated in Fig. 5. Alternatively, the production apparatus illustrated in Fig. 6 is used to implement the collagen yarn obtaining step S20 as well as the collagen yarn drawing step S30. In the following, the general outline of the production apparatus will be described, and also the steps of the above-mentioned method of producing a collagen yarn will be specifically described.

### <Production Apparatus Used for Preparing Collagen Yarn>

Referring to Fig. 5, a production apparatus 100 to be used for preparing collagen yarn 1 has a vessel 101 for accommodating the neutral collagen solution, a needle 102 as a channel through which the neutral collagen solution is discharged from vessel 101 to the outside, and a driving apparatus 103 (a syringe pump, for example) that generates the shear rate for discharging the neutral collagen solution through the needle 102 to the outside. Production apparatus 100 further has a coagulation bath 104 that accommodates the neutral collagen solution discharged to the outside. Coagulation bath 104 is filled with an ethanol solution having a concentration from 70 volume% to 95 volume%. As described above, the collagen in the neutral collagen solution is discharged through the needle 102 into coagulation bath 104, and becomes deposited as collagen yarn 1 in the ethanol solution.

Vessel 101 is not particularly limited in terms of its shape and the like, as long as it functions to hold the neutral collagen solution and discharge the neutral collagen solution through the needle 102 to the outside. Preferably, vessel 101 includes a syringe for holding the neutral collagen solution, for example, and a plunger driven by the driving apparatus 103 to discharge the neutral collagen solution from the syringe through the needle 102 to the outside. In this case, it is possible to easily install needle 102 to the syringe, in the same manner as for a so-called injector. Each of the syringe and the plunger can have a suitable size for the amount of the neutral collagen solution to accommodate. Each of the syringe and the plunger may be made of resin, glass, or metal. As driving apparatus 103, various pumps and the like capable of sucking and transferring liquid, gas, and the like by the action of pressure to achieve the above-mentioned functions can be used. For instance, in the case where the present production method is applied to industry, a pneumatic driving apparatus and/or the like may be used.

The inner diameter of needle 102 is from 100 µm to 300 µm. Preferably, the inner diameter of needle 102 is from 125 µm to 250 µm. In this case, in the collagen yarn obtaining step S20, the shear rate of the neutral collagen solution discharged through the needle 102 can be properly adjusted by the driving apparatus 103, and thereby collagen yarn 1 having a desired diameter can be obtained. The shear rate of the neutral collagen solution discharged through the needle 102 is from 100 s⁻¹ to 1500 s⁻¹. From the viewpoint of easily adjusting the diameter of the collagen yarn, the shear rate is preferably from 300 s⁻¹ to 1000 s⁻¹. When the radius of needle 102 is defined as r (mm) and the flow speed at which the neutral collagen solution is discharged is defined as Q (mm³·s⁻¹), the shear rate is 4Q/πr³ (unit, s⁻¹) (where π represents the circular constant). It should be noted that when the collagen concentration of the neutral collagen solution is high and the inner diameter of the needle is small, the actual Q may be below the input value, depending on the performance of the driving apparatus. In that case, it may be necessary to find conditions where the take-up speed of a take-up roller 105 is balanced with the discharge speed of the collagen yarn 1, to determine the actual Q.

Referring to Fig. 6, for implementation of the collagen yarn drawing step S30, production apparatus 100 preferably comprises the take-up roller 105 for taking up the collagen yarn 1 deposited in the ethanol solution in the coagulation bath 104, as well as a motor 106 for driving the take-up roller 105. Further preferably, production apparatus 100 has a relay roller 107 that is placed between the coagulation bath 104 and the take-up roller 105 to relay the collagen yarn 1 from the ethanol solution to the take-up roller 105. Equipped with relay roller 107, during the take-up process, production apparatus 100 is capable of taking up the collagen yarn 1 on take-up roller 105 without entanglement. As take-up roller 105, motor 106, and relay roller 107, conventionally known rollers and motors can be used as long as they have necessary functions.

### <Steps of Method of Producing Collagen Yarn>

### (First Step, Preparation Step)

The method of producing a collagen yarn according to the present embodiment includes Step S10 to prepare the neutral collagen solution containing collagen in a concentration from 3.0 mass% to 5.0 mass%. The collagen is atelocollagen. Preparation Step S10 aims at preparing a neutral collagen solution to serve as a raw material of collagen yarns 1.

The neutral collagen solution can be prepared by mixing an acidic atelocollagen solution having a concentration higher than the target concentration, with a neutral solvent (for example, a salt-containing solvent such as phosphate buffer physiological saline) by a conventionally known method, so that the atelocollagen concentration falls within the range of 3.0 mass% to 5.0 mass%. In the case when the neutral collagen solution is discharged toward the coagulation bath filled with an ethanol solution having a concentration from 70 volume% to 95 volume% in the below-described collagen yarn obtaining step S20, collagen molecules form fibrils in an instant in the coagulation bath and thereby collagen yarns 1 can be obtained. As described above, the pH of the neutral collagen solution is preferably from 6.0 to 9.0. The neutral collagen solution prepared in this step is accommodated in vessel 101 (a syringe, in particular) of production apparatus 100. Preferably, air bubbles are removed from the neutral collagen solution by a conventionally known method with the use of a centrifuge and/or the like. If air bubbles remain, collagen yarns can break at the time of production and thereby the yield can be impaired.

When the collagen concentration of the neutral collagen solution is less than 3.0 mass%, collagen molecules may not easily form fibrils in an instant in the coagulation bath in the collagen yarn obtaining step S20, and thereby desired collagen yarns 1 may not be obtained. When the collagen concentration of the neutral collagen solution is more than 5.0 mass%, even when the liquid temperature is maintained at 10°C or less, collagen may form fibrils in the neutral solvent and thereby desired collagen yarns 1 may not be obtained. Preferably, the neutral collagen solution contains collagen (atelocollagen, in particular) in a concentration from 4.5 mass% to 5.0 mass%.

### (Second Step: Collagen Yarn Obtaining Step)

The method of producing a collagen yarn according to the present embodiment includes Step S20 to obtain collagen yarns by discharging the neutral collagen solution into an ethanol solution having a concentration from 70 volume% to 95 volume%. In the collagen yarn obtaining step S20, the neutral collagen solution is discharged through a channel having an inner diameter from 100 µm to 300 µm into the ethanol solution at a shear rate from 100 s⁻¹ to 1500 s⁻¹. The collagen yarn obtaining step S20 aims at passing the collagen molecules contained in the neutral collagen solution through the thin channel to make them aligned in the flowing direction, and, in that state, discharging them into the ethanol solution to form them into fibrils in an instant for obtaining collagen yarns 1.

Specifically, the collagen yarn obtaining step S20 is implemented at production apparatus 100 as described above. In the collagen yarn obtaining step S20, firstly, the neutral collagen solution accommodated in vessel 101 of production apparatus 100 is extruded by a plunger driven by the driving apparatus 103 that generates a shear rate of 100 s⁻¹ to 1500 s⁻¹. As a result, the neutral collagen solution is discharged from vessel 101, through the needle 102 having an inner diameter from 100 µm to 300 µm, toward the coagulation bath 104. Furthermore, in coagulation bath 104, collagen (atelocollagen) in the neutral collagen solution form fibrils in an instant which become deposited as collagen yarns 1.

When the concentration of the ethanol solution is less than 70 volume%, atelocollagen in the neutral collagen solution may not easily form fibrils in an instant in coagulation bath 104. When the concentration of the ethanol solution is more than 95 volume%, dehydration of the discharged neutral collagen solution proceeds too fast and thereby the resulting yarns tend to break easily. When the inner diameter of needle 102 in production apparatus 100 is less than 100 µm, too much pressure is required for extrusion and thereby a desired shear rate may not be obtained, and/or the flow of the neutral collagen solution immediately after discharged from the channel may become unstable and thereby the resulting yarns may break. When the inner diameter of needle 102 is more than 300 µm, collagen yarns having desired small diameters may not be obtained, and/or drying during the taking-up process may become difficult.

Furthermore, when the shear rate is less than 100 s⁻¹ in the collagen yarn obtaining step S20, the collagen molecules in the neutral collagen solution may not be aligned in the flowing direction when they are discharged into the ethanol solution. When the shear rate is more than 1500 s⁻¹, the die swell effect may occur and the flow of the neutral collagen solution discharged from the channel may become swollen, and for this and other reasons, collagen molecules in the neutral collagen solution may not be aligned in the flowing direction when they are discharged into the ethanol solution. Preferably, the shear rate is from 300 s⁻¹ to 1000 s⁻¹.

In the collagen yarn obtaining step S20, lastly, collagen yarns 1 can be taken out from coagulation bath 104 and taken up while being dried. Collagen yarns 1 taken out from coagulation bath 104 are wet with the ethanol solution, but the ethanol solution will be immediately volatilized in air. Therefore, collagen yarns 1 can be dried easily. In collagen yarns 1 obtained by the above-mentioned production method, the collagen nanofibrils can be arranged in a highly-aligned state all the way into the interior of collagen yarns 1, preferably into the core portion. Furthermore, according to the above-mentioned production method, in principle, the length of collagen yarns 1 is not limited. In addition, with the inner diameter of needle 102 and the shear rate properly adjusted, the diameter of collagen yarns 1 can be from 10 µm to 50 µm. With collagen yarns 1 made of atelocollagen, safety for living body can also be ensured. As a result, collagen yarns capable of artificially mimicking biological collagen fibers can be obtained.

### (Third Step, Collagen Yarn Drawing Step)

Preferably, the method of producing a collagen yarn according to the present embodiment further includes Step S30 to draw the collagen yarns by taking up the collagen yarns at a take-up speed of 1.5 times to 4.0 times the discharging linear speed. The collagen yarn drawing step S30 aims at making the collagen yarns 1 as thin as biological collagen fibers, enhancing the alignment of the collagen fibrils, and increasing the production speed of the collagen yarns.

Specifically, the collagen yarn drawing step S30 is implemented at production apparatus 100 illustrated in Fig. 6. More specifically, at production apparatus 100, collagen yarns 1 deposited in coagulation bath 104 are relayed by relay roller 107 to be taken up on take-up roller 105 driven by motor 106. At this time, the take-up speed for taking up the collagen yarns 1 on take-up roller 105 is preferably 1.5 times to 4.0 times the discharging linear speed at which the neutral collagen solution is discharged from the vessel 101 through the needle 102 toward the coagulation bath 104. More preferably, the take-up speed is 2.0 times to 3.5 times the discharging linear speed. In this case, at the same time as the collagen in the neutral collagen solution is becoming deposited and spun into collagen yarns 1 in coagulation bath 104, the collagen yarns 1 can be drawn, and thereby the diameter of the collagen yarns 1 can become as small as 30 µm or less, for example. Furthermore, the total length of collagen yarns 1 can become 1×10⁶ times or more the diameter, for example. As a result, thinner, longer collagen yarns 1 capable of artificially mimicking biological collagen fibers can be obtained. The take-up speed for taking up the collagen yarns 1 can be calculated from the speed of rotation of the motor and the diameter of the roller. The discharging linear speed for discharging the neutral collagen solution is determined by the equation Q/πr² (the unit is mm·s⁻¹=3.6 m/h), where Q (mm³·s⁻¹) represents the flow speed at which the neutral collagen solution is discharged and r (mm) represents the radius of the channel. It should be noted that it may be necessary to determine the actual Q, as described above.

### [Examples]

In the following, a more detailed description will be given of the present invention by way of examples, but these examples are not intended to limit the scope of the present invention. In these examples, to prepare collagen yarns of respective Samples, the inventors of the present invention used a production apparatus as illustrated in Fig. 5 or Fig. 6. In the below description, Sample 1 to Sample 6 are examples of collagen yarns within the scope of the present invention, and Sample 11 to Sample 19 are comparative examples.

### [Preparation of Collagen Yarn]

### <Sample 1>

### (First Step, Preparation Step)

Firstly, an acidic atelocollagen solution (under the trade name "Collagen BM", manufactured by Nitta Gelatin Inc., with a collagen concentration of 0.571%) was prepared. In addition, for neutralizing the acidic atelocollagen solution, a buffer (pH7; 12×NPB) having a concentration 12 times a 50-mM sodium hydrogen phosphate buffer containing sodium chloride in 140 mM (pH7; hereinafter called 1×NPB) was prepared. The acidic atelocollagen solution was concentrated with an evaporator at 29°C to a concentration within the range of 2 to 8 mass% to produce a concentrated collagen solution, and the accurate concentration of the resulting concentrated collagen solution was determined from the dry weight of the acidic atelocollagen solution. With the blending ratio between the concentrated collagen solution, pure water, and 12×NPB adjusted as appropriate, the neutral collagen solution specified in Table 1 was prepared in such a manner that the buffer concentration became 1×NPB. Thus, a neutral collagen solution containing collagen in a concentration of 4.8 mass% was prepared. It should be noted that because of the poor fluidity and the small amount, most of the neutral collagen solutions are difficult to directly measure the pH. For this reason, a solution (a hypothetical solution) was prepared by replacing the concentrated collagen solution with a pH3 dilute hydrochloric acid, followed by using this hypothetical solution together with pure water and 12×NPB in the blending ratio adjusted as described above, to prepare a pH-measurement solution, and the pH of the resultant was measured with a pH meter. At this time, the pH of the pH-measurement solution was checked to make sure that it was within the range of 7.1 to 7.4.

### (Second Step, Collagen Yarn Obtaining Step)

Then, the neutral collagen solution in the vessel was discharged through a needle having an inner diameter of 182 µm, toward the coagulation bath 104 filled with a 90-volume% ethanol solution (see Fig. 5). The shear rate at this time was set at 1000 s⁻¹. This condition was equivalent to a discharging linear speed of 82 m/h. In the coagulation bath, collagen (atelocollagen) in the neutral collagen solution formed fibrils in an instant to be deposited as collagen yarns, which became settled in the coagulation bath. Subsequently, the collagen yarns were taken up on a take-up roller. At this time, during the process from discharging to taking up, the collagen yarns did not undergo damage such as breakage, and they were rated as having good spinning capabilities. In this manner, collagen yarns of Sample 1 were obtained.

### <Sample 2 to Sample 4>

Except that the collagen solutions (neutral collagen solutions) specified in Table 1 were prepared and the inner diameter of the needle of the production apparatus, the shear rate, and the discharging linear speed were set as specified in Table 1, the same manner as in the preparation of the collagen yarns of Sample 1 was adopted to obtain collagen yarns of Sample 2 to Sample 4. During the process from discharging to taking up, the collagen yarns of Sample 2 to Sample 4 did not undergo damage such as breakage, and they were rated as having good spinning capabilities.

### <Sample 5>

The collagen solution (neutral collagen solution) specified in Table 1 was prepared, and the inner diameter of the needle of the production apparatus, the shear rate, and the discharging linear speed were set as specified in Table 1. Furthermore, the collagen yarns deposited in the coagulation bath were taken up on a take-up roller driven by a motor equipped in the production apparatus. The take-up speed for taking up the collagen yarns on the take-up roller at this time was set at 2.0 times the discharging linear speed for discharging the neutral collagen solution from the vessel through the needle toward the coagulation bath, and at the same time as the discharged collagen solution was being formed into yarns, the collagen was drawn (see Fig. 6). Except these, the same manner as in the preparation of the collagen yarns of Sample 1 was adopted to obtain collagen yarns of Sample 5. During the process from discharging to taking up, the collagen yarns of Sample 5 did not undergo damage such as breakage, and they were rated as having good spinning capabilities.

### <Sample 6>

Except that the take-up speed for taking up the collagen yarns on a take-up roller was set at 2.5 times the discharging linear speed for discharging the neutral collagen solution from the vessel through the needle toward the coagulation bath, the same manner as in the preparation of the collagen yarns of Sample 5 was adopted to obtain collagen yarns of Sample 6. During the process from discharging to taking up, the collagen yarns of Sample 6 did not undergo damage such as breakage, and they were rated as having good spinning capabilities.

### <Sample 11 to Sample 12>

Except that the collagen solutions (neutral collagen solutions) specified in Table 1 were prepared and the inner diameter of the needle of the production apparatus, the shear rate, and the discharging linear speed were set as specified in Table 1, the same manner as in the preparation of the collagen yarns of Sample 1 was adopted to obtain collagen yarns of Sample 11 to Sample 12. Referring to Table 1, in the preparation of the collagen yarns of Sample 11 to Sample 12, the shear rate was set at 2000 s⁻¹ or more. During the process from discharging to taking up, these collagen yarns did not undergo damage such as breakage, and they were rated as having good spinning capabilities.

### <Samples 13 to 15>

Referring to the production method described in PTL 1, Samples 13 to 15 were obtained. Specifically, except that the collagen concentration of the neutral collagen solution was set at 2.5%, a needle having a diameter of 521 µm was used, the shear rate and the discharging linear speed were set as specified in Table 1, and ethanol for the coagulation bath was changed to a 10-mM phosphate buffer (pH7.0; warmed to 37°C), the same manner as in the preparation of the collagen yarns of Sample 1 was adopted to obtain collagen yarns of Sample 13. Except that the collagen concentration of the neutral collagen solution was set at 5.0%, the same manner as for Sample 13 was adopted to obtain collagen yarns of Sample 14. Except that the collagen concentration of the neutral collagen solution was set at 2.5% and the coagulation bath filled with phosphate buffer was followed by another one filled with 90% ethanol, the same manner as for Sample 14 was adopted to obtain collagen yarns of Sample 15. Even though the spinning rate was low, Samples 13 and 15 were rated as having good spinning capabilities. On the other hand, the collagen yarns of Sample 14 frequently broke during the taking-up process, and therefore they were rated as having poor spinning capabilities.

### <Sample 16 to Sample 19>

Except that the collagen solutions (pH3 acidic collagen solutions) specified in Table 1 were prepared and the inner diameter of the needle of the production apparatus, the shear rate, and the discharging linear speed were set as specified in Table 1, the same manner as in the preparation of the collagen yarns of Sample 1 was adopted to obtain collagen yarns of Sample 16 to Sample 19. Referring to Table 1, in the preparation of the collagen yarns of Sample 16 and Sample 17, the inner diameter of the needle was set at 1500 µm and the shear rate was set at 20 s⁻¹. In the preparation of the collagen yarns of Sample 16, the collagen concentration of the acidic collagen solution was set at 0.6 mass%, and in the preparation of the collagen yarns of Sample 17, the collagen concentration of the acidic collagen solution was set at 5.0 mass%. In the preparation of the collagen yarns of Sample 18, the collagen concentration of the acidic collagen solution was set at 0.6 mass% and the shear rate was set at 20 s⁻¹. Sample 16 to Sample 19 were produced as reproducibility experiment of prior-art wet spinning.

As a result, as for Sample 16, fusion occurred in the collagen solution discharged into the coagulation bath leading to precipitation, and as a result, spinning did not occur and collagen yarns were not formed (poor spinning capabilities). As for Sample 17, only the surface of the collagen yarns turned cloudy and whitish in the coagulation bath and therefore it was presumed that fibril formation occurred, but the interior remained transparent for several minutes (poor spinning capabilities). As for Sample 18, fusion occurred in the coagulation bath to form lumps before the collagen formed yarns, and spinning did not occur (poor spinning capabilities). The collagen yarns of Sample 19 turned translucent and became settled at the bottom of the coagulation bath, and after this, they were taken up successfully (good spinning capabilities).

The overview of the preparation of the collagen yarns of Sample 1 to Sample 6 and Sample 11 to Sample 19 is given in Table 1 (the collagen solution, the inner diameter of the needle, the shear rate, the discharging linear speed, the spinning capabilities, and the like).

### [Table 1]

**Table. 1**

| | Collagen solution | | | Needle inner diameter [µm] | Shear rate [s⁻¹] | Take-up speed/ discharging linear speed | Discharging linear speed [m/h] | Spinning capabilities |
|---|---|---|---|---|---|---|---|---|
| | Raw material | Conc. [mass%] | pH | | | | | |
| Sample 1 | Atelo | 4.8 | 7 | 182 | 1000 | - | 82 | G |
| Sample 2 | Atelo | 4.8 | 7 | 182 | 300 | - | 25 | G |
| Sample 3 | Atelo | 5.0 | 7 | 134 | 300 | - | 18 | G |
| Sample 4 | Atelo | 4.5 | 7 | 134 | 800 | - | 48 | G |
| Sample 5 | Atelo | 4.5 | 7 | 211 | 300 | 2.0 | 57 | G |
| Sample 6 | Atelo | 4.5 | 7 | 211 | 300 | 2.5 | 71 | G |
| Sample 11 | Atelo | 4.8 | 7 | 182 | 2000 | - | 160 | G |
| Sample 12 | Atelo | 4.8 | 7 | 182 | 5000 | - | 410 | G |
| Sample 13 | Atelo | 2.5 | 7 | 521 | 20 | - | 4.6 | G |
| Sample 14 | Atelo | 5.0 | 7 | 182 | 20 | - | 3.0 | NG |
| Sample 15 | Atelo | 2.5 | 7 | 182 | 20 | - | 3.0 | G |
| Sample 16 | Atelo | 0.6 | 3 | 1500 | 20 | - | 14 | NG |
| Sample 17 | Atelo | 5.0 | 3 | 1500 | 20 | - | 14 | NG |
| Sample 18 | Atelo | 0.6 | 3 | 182 | 20 | - | 3.0 | NG |
| Sample 19 | Atelo | 7.0 | 3 | 211 | 1000 | - | 95 | G |

### [Evaluation of Collagen Yarns]

As for the collagen yarns of Sample 1 to Sample 6, Sample 11 to Sample 13, Sample 15, and Sample 19, by the above-described methods, the diameter (average value and standard deviation), the total length, and the refractive index difference (average value and standard deviation) were determined. Furthermore, as for the collagen yarns of Sample 1 to Sample 6, Sample 11 to Sample 13, Sample 15, and Sample 19, by the following method, Young's modulus (average value and standard deviation) was measured to evaluate strength. Results are given in Table 2. The collagen yarns of Sample 14 and Sample 16 to Sample 18 were impossible to take up, and therefore evaluation of the above-listed items were not performed.

Young's modulus was calculated from the initial slope of a stress-strain curve obtained by a tensile test. The collagen yarn was cut into about 40 mm, and both ends were fixed with plastic tape so as to ensure a sample length of 20 mm. The collagen yarn was fixed to the chucks (spaced by 20 mm) of a strength tester (under the trade name "TA.XTplus", manufactured by Stable Micro Systems), and strength testing was performed at a deformation rate of 1 mm/second to produce a load-deformation curve. The load was divided by the cross-sectional area (average value of n=5) of the collagen yarn separately measured with a scanning electron microscope, to determine the stress, and the deformation (mm) was divided by the initial chuck-to-chuck distance (mm) to determine the strain, followed by converting the load-deformation curve to a stress-strain curve. From the slope of the straight line for strain of 0.002 to 0.008 on this curve, Young's modulus was calculated (n=5). When Young's modulus was 2.0 GPa or more, the collagen yarn was evaluated as being strong enough for use as a biological material.

### [Table 2]

**Table. 2**

| | Diameter [µm] | | Total length [m] | Refractive index difference [δn×10⁴] | | Young's modulus [GPa] | |
|---|---|---|---|---|---|---|---|
| | mean | SD | | mean | SD | mean | SD |
| Sample 1 | 45.0 | 2.4 | >10 | 7.12 | 0.10 | 2.70 | 0.58 |
| Sample 2 | 37.6 | 1.6 | >10 | 5.82 | 0.03 | 3.34 | 0.21 |
| Sample 3 | 27.8 | 0.7 | >10 | 5.80 | 0.26 | 2.32 | 0.37 |
| Sample 4 | 23.1 | 0.7 | >10 | 4.36 | 0.16 | 2.74 | 0.29 |
| Sample 5 | 28.3 | 1.4 | >10 | 10.7 | 0.70 | 2.68 | 0.26 |
| Sample 6 | 26.8 | 1.5 | >10 | 11.3 | 0.30 | 2.75 | 0.15 |
| Sample 11 | 52.7 | 1.6 | >10 | 3.81 | 0.05 | 3.38 | 0.21 |
| Sample 12 | 59.1 | 0.8 | >10 | 1.01 | 0.16 | 3.60 | 0.23 |
| Sample 13 | 67.6 | 1.8 | 0.2-0.5 | 3.03 | 0.16 | 3.61 | 0.21 |
| Sample 15 | - | - | >10 | 0.27 | 0.01 | - | 0.23 |
| Sample 19 | 50.7 | 0.5 | >10 | 0.84 | 0.20 | 1.74 | 0.15 |

### [Discussion]

Each of the collagen yarns of Sample 1 to Sample 6 had a diameter of 10 µm to 50 µm and a total length of 2×10⁵ times or more the diameter. The refractive index difference was 4.3×10⁻⁴ or more, and they were evaluated that the collagen nanofibrils constituting the collagen yarns were arranged in a highly-aligned state along the longitudinal direction of the collagen yarns. Furthermore, Young's modulus was 2.0 GPa or more, and therefore the collagen yarns were evaluated as being strong enough for use as a biological material. This numerical value, 2.0 GPa, is equivalent to the elastic modulus of a collagen yarn obtained by traditional wet spinning, a method of spinning in which collagen is sufficiently formed into fibrils in a neutral coagulation bath. From the above description, it was suggested that the collagen yarns of Sample 1 to Sample 6 artificially mimicked biological collagen fibers and were useful as a biological material such as an artificial tendon or a membrane material.

On the other hand, each of the collagen yarns of Sample 11 to Sample 12 had a diameter of more than 50 µm and a refractive index difference of less than 4.3×10⁻⁴. The collagen yarns of Sample 13 had a diameter of more than 50 µm, a total length of 0.2 to 0.5 m, and a refractive index difference of less than 4.3 ×10⁻⁴. The collagen yarns of Sample 15 noticeably curled at the time of drying and were obviously not useful for a biological material, and therefore, the diameter was not determined and the tensile test was not performed. The collagen yarns of Sample 15 had a refractive index difference of less than 4.3×10⁻⁴. The collagen yarns of Sample 19 had a diameter of more than 50 µm and a refractive index difference of less than 4.3×10⁻⁴. The collagen yarns of Sample 14 and Sample 16 to Sample 18 were impossible to take up in the form of yarns. Therefore, the collagen yarns of Sample 11 to Sample 19 were not rated as being capable of artificially mimicking biological collagen fibers.

The embodiments and examples of the present invention are described above, and it is originally planned that the configurations of the above embodiments and examples may be combined as appropriate.

It should be construed that the embodiments and examples disclosed herein are given by way of illustration in all respects, not by way of limitation. It is intended that the scope of the present invention is defined by claims, not by the above description, and encompasses all modifications and variations equivalent in meaning and scope to the claims.

### REFERENCE SIGNS LIST

1 collagen yarn, 11 collagen nanofibril, 10 hydrated collagen yarn, 200 artificial tendon, 100 production apparatus, 101 vessel, 102 needle (channel), 103 driving apparatus, 104 coagulation bath, 105 take-up roller, 106 motor, 107 relay roller.

## Claims

1. A collagen yarn comprising collagen nanofibrils, wherein
the collagen yarn has a diameter from 10 µm to 50 µm,
the collagen yarn has a total length of 2×10⁵ times or more the diameter,
the collagen nanofibrils are arranged in a highly-aligned state along a longitudinal direction of the collagen yarn,
the highly-aligned state is a state where birefringence measurement of a hydrated collagen yarn yields a refractive index difference of 4.3×10⁻⁴ or more, and
the hydrated collagen yarn is obtained by immersing the collagen yarn in a neutral phosphate buffer.

2. The collagen yarn according to claim 1, wherein the collagen nanofibrils are made of atelocollagen.

3. The collagen yarn according to claim 1 or 2, wherein the total length is 1×10⁶ times or more the diameter.

4. A method of producing a collagen yarn, the method comprising:
preparing a neutral collagen solution containing collagen in a concentration from 3.0 mass% to 5.0 mass%; and
obtaining a collagen yarn by discharging the neutral collagen solution into an ethanol solution having a concentration from 70 volume% to 95 volume%, wherein
the collagen is atelocollagen, and
in the obtaining a collagen yarn, the neutral collagen solution is discharged through a channel having an inner diameter from 100 µm to 300 µm into the ethanol solution at a shear rate from 100 s⁻¹ to 1500 s⁻¹.

5. The method of producing a collagen yarn according to claim 4, further comprising drawing the collagen yarn by taking up the collagen yarn at a take-up speed of 1.5 times to 4.0 times a discharging linear speed.

6. The method of producing a collagen yarn according to claim 4 or 5, wherein the neutral collagen solution contains the collagen in a concentration from 4.5 mass% to 5.0 mass%.

7. The method of producing a collagen yarn according to any one of claims 4 to 6, wherein the shear rate is from 300 s⁻¹ to 1000 s⁻¹.

8. A biological material containing the collagen yarn according to any one of claims 1 to 3.

9. The biological material according to claim 8, wherein the biological material is an artificial tendon or a membrane material.
